# EUROPÄISCHE PATENTSCHRIFT

(11) **EP 1 350 478 B1**
(45) Veröffentlichungstag und Bekanntmachung des Hinweises auf die Patenterteilung: **26.12.2007**
(21) Anmeldenummer: 03002409.5
(22) Anmeldetag: 04.02.2003
(51) Int. Cl.: A61B 17/58, A61F 2/46

(54) **Kanüle zum Applizieren von medizinischen Stoffen**
Cannula for application of medical substances
Cannule pour l'application de matières médicales

(30) Priorität: 05.04.2002 DE 10215191
(43) Veröffentlichungstag der Anmeldung: 08.10.2003
(73) Patentinhaber: Pajunk GmbH & Co. KG Besitzverwaltung, 78187 Geisingen (DE)
(72) Erfinder: Pajunk, Horst, 78187 Geisingen (DE); Pajunk, Heinrich, 78187 Geisingen-Kirchen-Hausen (DE)
(74) Vertreter: Manitz, Finsterwald & Partner GbR

(56) Entgegenhaltungen:
- EP-A- 0 356 810
- EP-A- 0 529 341
- EP-A- 0 608 985
- WO-A-02/02033
- DE-A- 3 102 142
- DE-A- 10 064 202
- DE-U- 9 303 961
- US-A- 5 304 141

## Beschreibung

Die vorliegende Erfindung betrifft ein Knochenzement-Applikationssystem umfassend eine Kanüle mit einem innenliegenden Hohlraum, der sich von einer am distalen Ende der Kanüle ausgebildeten distalen Kanülenöffnung zu einer am proximalen Ende der Kanüle ausgebildetem proximalen Kanülenöffnung erstreckt, sowie mit einem Mandrin, der in dem Hohlraum längsverschiebbar angeordnet ist.

Ein Applikationssystem der eingangs genannten Art ist aus der DE 100 64 202 A bekannt. In dieser deutschen Offenlegungsschrift ist eine Vorrichtung zum Applizieren von Knochenzement sowie eine Kanüle für eine solche Vorrichtung beschrieben, mit denen die Applikation des Knochenzements in kurzer Zeit durchgeführt werden kann, wobei gleichzeitig der für das Einbringen von Knochenzement erforderliche hohe Druck aufgebaut wird und eine Steuerbarkeit des applizierten Knochenzements möglich ist. Diese Vorrichtung wird beispielsweise verwendet, wenn Knochenstrukturen z.B. durch Knochenkrebs oder durch Osteoporose zersetzt oder brüchig geworden sind. Mit einer entsprechenden Vorrichtung ist die Applikation von Knochenzement unmittelbar in die betroffenen Knochenstrukturen möglich, wodurch diese verfestigt werden.

Die bekannte Vorrichtung hat sich in der Praxis bewährt. Beim Einsatz dieser Vorrichtung zeigt sich jedoch, dass aufgrund des relativ großen Durchmessers der Applikationskanüle die beim Einführen der Kanüle vom Patienten empfundenen Schmerzen relativ hoch sein können. Aufgrund des großen Durchmessers der Kanüle ist auch die exakte Platzierung der Kanüle relativ schwierig.

Aufgabe der Erfindung ist es, ein Applikationssystem der eingangs genannten Art anzugeben, mit der eine perkutane Vertebroplastie in einer maximal schonenden Art, d.h. mit möglichst geringen Schmerz für den Patienten und minimaler Verletzung des Gewebes durchgeführt werden kann. Dabei soll gleichzeitig eine möglichst exakte Platzierung der Kanüle möglich sein.

Diese Aufgabe wird durch ein Applikationssytem mit den Merkmalen des Anspruchs 1 gelöst.

US 5.304.141 A beschreibt eine Vorrichtung mit einer Epiduralnadel, einem in die Epiduralnadel einsetzbaren Inserter und einer in den Inserter einsetzbaren Spinalnadel. Die Spinalnadel umfasst einen Schaft, welcher einen Injektionskanal aufweist, durch den ein Narkosemittel in den Spinalkanal einer Wirbelsäule injizierbar ist. Der Schaft ist dazu an einem Basisstück angebracht, das mit einer Spritze verbindbar ist. Anstelle der Spinalnadel kann auch eine Kombination aus einem Katheter und einem Mandrin vorgesehen sein. EP 0 608 985 A1 beschreibt ebenfalls eine Epiduralnadel in welche ein Inserter einsetzbar ist, an dem allerdings die Spiralnadel fest angebracht ist.

EP 0 529 341 A1 beschreibt eine Epiduralkanüle, in der ein Mandrin platziert ist, der mit einer sich über seine Länge erstreckenden Aussparung versehen ist. Die Aussparung fluchtet mit einerDurchtrittsöffnung in der Epiduralkanüle, so dass eine durch die Aussparung vorgeschobene Spinalkanüle zwangsläufig mit ihrer Spitze der Durchtrittsöffnung zugeleitet wird und durch diese unbehindert hindurchtreten kann, um schließlich in einen Spinalkanal zu gelangen.

Der die Injektionskanüle betreffende Teil der Aufgabe wird erfindungsgemäß dadurch gelöst, dass das Anschlusselement von der Injektionsnadel abnehmbar und an dieser wieder befestigbar ausgebildet ist.

Gemäß der Erfindung ist somit der Mandarin der Applikationskanüle nicht, wie üblich, als Vollmaterial ausgebildet, sondern mit einer sich in Längsrichtung erstreckenden Bohrung versehen. Durch diese Bohrung kann eine Injektionsnadel geführt werden, so dass während des Einführens der Kanüle ständig Anästhesiemittel eingespritzt werden kann. Dadurch werden die Schmerzen beim Einführen der Kanüle verringert bzw. ausgeschaltet. Je nach Anwendung kann die Injektionskanüle dabei gleichzeitig eine Art Führungsdraht für die Applikationskanüle bilden, so dass das Einbringen der relativ dicken Applikationskanüle über die bereits eingesetzte Injektionskanüle erleichtert wird.

Grundsätzlich sind die zwei folgenden Anwendungsmöglichkeiten denkbar: Zum einen ist es möglich, zunächst die Injektionskanüle bis an die Cortex des zu behandelnden Knochens bzw. Wirbelkörpers einzuführen, wobei während des gesamten Einführvorganges Anästhesiemittel eingespritzt wird. Auf diese Weise wird erreicht, dass das zwischen der Hautoberfläche und dem Knochen liegende Gewebe über den gesamten Einführbereich anästhesiert ist, wenn anschließend die auf die Injektionskanüle aufgesetzte Applikationskanüle mit Hohlmandrin über die Injektionskanüle zu dem zu behandelnden Knochen eingebracht wird.

Zum anderen ist es auch möglich, dass die Injektionskanüle zunächst relativ gering in das Gewebe eingestochen und Anästhesiemittel eingespritzt wird, wodurch im Bereich des distalen Endes der Injektionskanüle eine örtliche Betäubung erfolgt. Anschließend wird die Applikationskanüle mit dem eingesetzten Mandrin auf die eingesetzte Injektionskanüle aufgeschoben und beide Kanülen werden nun zusammen weiter eingeführt, wobei jeweils das distale Ende der Injektionskanüle dem distalen Ende der Applikationskanüle vorausläuft, so dass durch das ständige Einspritzen von Anästhesiemittel jeweils der gerade die distalen Enden der beiden Kanülen umgebende Gewebebereich örtlich betäubt ist.

In beiden Fällen ist gewährleistet, dass eine maximal schonende Einführung der relativ dicken Applikationskanüle gegeben ist und die Schmerzempfindung des Patienten verringert bzw. ausgeschaltet ist.

Bei einer weiteren vorteilhaften Ausführungsform der Erfindung ist an dem proximalen Ende des Mandrins ein Abschlusselement ausgebildet, wobei die proximale Mandrinöffnung in dem Abschlusselement ausgebildet ist und der Führungskanal durch das Abschlusselement hindurch zu der proximalen Mandrinöffnung verläuft. Das Abschlusselement kann dabei zum einen zum Halten des Mandrins beim Einsetzen in die Kanüle und beim Herausziehen aus der Kanüle verwendet werden. Zum anderen kann das Abschlusselement dazu verwendet werden, die Kanüle, falls erforderlich, mit einem Eintriebsmittel, beispielsweise einem Hammer, an die gewünschte Position zu verbringen.

Nach einer weiteren bevorzugten Ausführungsform der Erfindung ist der Mandrin in dem Hohlraum im Wesentlichen spielfrei längsverschiebbar. Beispielsweise können der Hohlraum und der Mandrin jeweils einen im Wesentlichen kreisförmigen Querschnitt besitzen, wobei der Querschnittsdurchmesser des Hohlraums dabei geringfügig größer ist als der äußere Querschnittsdurchmesser des Mandrins. Grundsätzlich können die Querschnittsformen jedoch auch eine von der Kreisform abweichende, beispielsweise eine ovale, mehreckige, insbesondere dreieckige, oder eine sonstige geeignete Form aufweisen. Wesentlich ist dabei nur, dass eine im Wesentlichen spielfreie Führung des Mandarins in dem Hohlraum der Kanüle gegeben ist, was beispielsweise durch eine komplementäre Ausbildung der jeweiligen Querschnittsformen erreichbar ist.

Auch der Führungskanal kann einen im Wesentlichen kreisförmigen Querschnitt besitzen, wobei in diesem Fall auch die Injektionskanüle bevorzugt einen kreisförmigen Außenquerschnitt aufweist. Auch hier ist es jedoch lediglich erforderlich, dass die Injektionskanüle im Wesentlichen spielfrei in dem Führungskanal geführt wird, was wiederum durch die bereits angegebenen Querschnittsformen und insbesondere durch eine komplementäre Ausbildung der Querschnittsformen erreicht werden kann.

Nach einer weiteren vorteilhaften Ausführungsform der Erfindung ist an dem proximalen Ende der Kanüle ein Kopplungsabschnitt zum Ankoppeln der Kanüle an die Vorrichtung zum Applizieren von medizinischen Stoffen vorgesehen. Das Kopplungselement kann dabei beispielsweise als große Luer Lock-Verbindung ausgebildet sein, das mit einem entsprechenden Gegenelement an der Applikationsvorrichtung abdichtend verbunden werden kann.

Vorteilhaft ist in dem Bereich des proximalen Endes der Kanüle, insbesondere an dem Kopplungsabschnitt, ein Verbindungselement zur Erzeugung einer lösbaren, drehfesten und/oder in axialer Richtung verschiebefesten Verbindung zwischen Mandrin und der Kanüle vorgesehen. Diese Verbindung kann beispielsweise als Bajonettverschluss ausgebildet sein.

Aufgrund der erfindungsgemäßen Ausbildung der Injektionskanüle ist es möglich, nach Einführen der Injektionskanüle zunächst das abnehmbare Anschlusselement von dem proximalen Ende der Injektionskanüle zu entfernen, so dass das proximale Ende der Injektionsnadel frei liegt. Auf diese Weise kann die Applikationskanüle mit ihrer distalen Kanülenöffnung über das proximale Ende der Injektionskanüle geschoben und so lange in das Gewebe eingeführt werden, bis das proximale Ende der Injektionsnadel aus der proximalen Kanülenöffnung austritt. Anschließend kann das Anschlusselement wieder auf das proximale Ende der Injektionsnadel aufgesetzt und an dieser wieder befestigt werden. An das Anschlusselement kann dann wiederum die Spritze mit Anästhesiemittel angeschlossen werden, so dass eine weitere Zuführung von Anästhesiemittel beim weiteren Einführen der Applikationskanüle möglich ist.

Nach einer weiteren bevorzugten Ausführungsform der erfindungsgemäßen Injektionskanüle ist bei abgenommenem Anschlusselement die Querschnittsaußenabmessung des proximalen Bereichs der Injektionsnadel im wesentlichen gleich der Querschnittsaußenabmessung des übrigen Bereichs der Injektionsnadel. Dadurch ist gewährleistet, dass beispielsweise aufgrund der Befestigung für das Anschlusselement keine Verdickungen am proximalen Ende der Injektionsnadel vorgesehen sind, die ein Auffädeln der Applikationskanüle mit Mandrin auf die Injektionsnadel über deren proximales Ende verhindern würden. Selbstverständlich ist von dem genannten übrigen Bereich der Injektionsnadel nicht die verjüngende Spitze der Injektionsnadel gemeint, deren Querschnittsaußenabmessung bis hin zum spitzenförmigen Ende ständig abnimmt und daher deutlich geringer ist als die Querschnittsaußenabmessung der restlichen Injektionsnadel.

Bevorzugt umfasst das Anschlusselement eine Klemmeinheit zur klemmbaren Befestigung des Anschlusselements an der Injektionsnadel. Dadurch ist eine besonders einfache und kostengünstige Befestigung des Anschlusselements an der Injektionsnadel möglich. Weiterhin wird erreicht, dass an der Injektionsnadel selbst keine eventuell nach außen vorstehenden Elemente zur Befestigung des Anschlusselements vorgesehen sein müssen.

Nach einer weiteren vorteilhaften Ausführungsform der Erfindung ist in dem Injektionskanal ein Injektionskanülenmandrin einsetzbar. Bevorzugt ist der Injektionskanülenmandrin dabei in dem Injektionskanal längsverschiebbar. Durch einen Injektionskanülenmandrin wird erreicht, dass beim Einstechen und weiteren Einführen der Injektionskanüle ein Verstopfen der Injektionskanüle durch Eindringen von Gewebe vermieden wird.

Insbesondere kann dabei das distale Ende des Injektionskanülenmandrins abgerundet, insbesondere halbkugelförmig ausgebildet sein. Bevorzugt ist der Injektionskanülenmandrin dabei so lang ausgebildet, dass bei vollständig in den Injektionskanal eingesetztem Injektionskanülenmandrin dessen distales Ende in Höhe der Injektionsöffnung zu liegen kommt. Durch diese Ausgestaltung ist auch bei gegeneinander verdrehbarer Injektionskanüle und Injektionskanülenmandrin gewährleistet, dass die Injektionsöffnung am distalen Ende der Injektionskanüle sicher abgedichtet ist, ohne dass durch eine eventuelle vorstehende Spitze des Injektionskanülenmandrins eine zusätzliche Verletzung des Gewebes erfolgen würde.

Nach einer weiteren bevorzugten Ausführungsform der Erfindung ist an dem proximalen Ende des Injektionskanülenmandrins ein Fixierelement vorgesehen, mit dem der Injektionskanülenmandrin an der Injektionskanüle in Längsrichtung unverschiebbar fixierbar ist. Auf diese Weise wird sichergestellt, dass das insbesondere abgerundete, distale Ende des Injektionskanülenmandrins exakt an der gewünschten Stelle in Höhe der Injektionsöffnung liegt.

Weitere vorteilhafte Ausführungsformen der Erfindung sind in den Unteransprüchen angegeben.

Die Erfindung wird nachfolgend anhand von Ausführungsbeispielen unter Bezugnahme auf die Zeichnungen näher beschrieben; in diesen zeigen:
- Fig. 1: eine erfindungsgemäß ausgebildete Kanüle,
- Fig. 2: eine Detailansicht der Kanüle nach Fig. 1,
- Fig. 3: eine erfindungsgemäß ausgebildete Injektionskanüle,
- Fig. 4: einen Injektionskanülenmandrin gemäß der Erfindung,
- Fig. 5: den in die Injektionskanüle nach Fig. 3 eingesetzten Injektionskanülenmandrin nach Fig. 4,
- Fig. 6: die Injektionskanüle nach Fig. 3 mit abgenommenem Anschlusselement und
- Fig. 7: die komplette Injektionskanüle nach Fig. 5 eingesetzt in die Kanüle nach den Fig. 1 und 2.

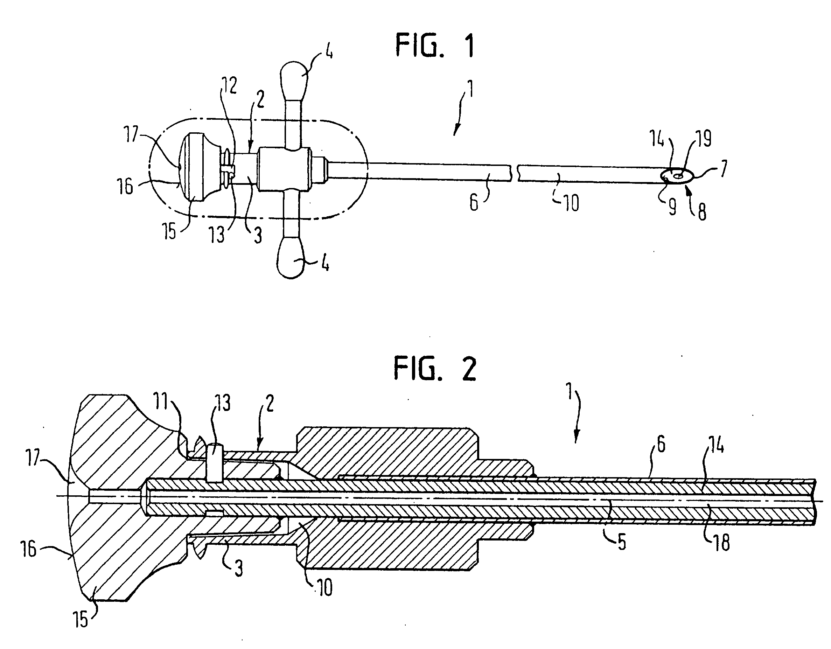

Fig. 1 zeigt eine erfindungsgemäß ausgebildete Kanüle 1 zum Applizieren von medizinischen Stoffen, wie beispielsweise Knochenzement. Die Kanüle 1 besitzt an ihrem proximalen Ende 2 einen beispielsweise als große Luer Lock-Verbindung ausgebildeten Kopplungsabschnitt 3, der mit einem entsprechenden Kopplungselement einer nicht dargestellten Vorrichtung zum Applizieren von Knochenzement abdichtend verbunden werden kann. Der Kopplungsabschnitt 3 ist beispielsweise aus Metall hergestellt, um die beim Einschlagen der Kanüle 1 auftretenden Kräfte aufnehmen zu können.

An dem proximalen Ende 2 der Kanüle 1 sind zwei sich radial nach außen erstreckende, stiftförmige Betätigungselemente 4 vorgesehen, über die die eingesetzte Kanüle 1 in einfacher Weise sowohl um ihre Längsachse 5 (Fig. 2) verdreht als auch in Richtung der Längsachse 5 aus dem Körper des Patienten wieder herausgezogen werden kann.

An das proximale Ende 2 der Kanüle 1 schließt sich ein Kanülenrohr 6 an, das proximalseitig in einer Spitze 7 endet. Dabei ist an dem distalen Ende 8 der Kanüle 1 eine distale Kanülenöffnung 9 ausgebildet, deren Querschnitt sich schräg zur Längsachse 5 der Kanüle 1 erstreckt. Die Außenkanten der distalen Kanülenöffnung 9 sind dabei als Schneiden ausgebildet.

Die distale Kanülenöffnung 9 bildet das Ende eines im Inneren des Kanülenrohrs 6 liegenden Hohlraums 10, der sich über die gesamte Länge der Kanüle 1 erstreckt und die distale Kanülenöffnung 9 mit einer in Fig. 1 nicht sichtbaren proximalen Kanülenöffnung 11 verbindet.

Im Bereich dieser proximalen Kanülenöffnung 11 ist an dem proximalen Ende 2 der Kanüle 1 weiterhin eine L-förmige Ausnehmung 12 ausgebildet, die zusammen mit einem stiftförmigen Ansatz 13 einen Bajonettverschluss bildet. Der stiftförmige Ansatz 13 ist dabei im Bereich des proximalen Endes eines Mandrins 14 angeordnet, der in den Hohlraum 10 der Kanüle 1 eingesetzt ist.

Der Mandrin 14 besitzt an seinem proximalen Ende ein Abschlusselement 15, das zum einen zum Halten des Mandrins 14 beim Einsetzen in die Kanüle 1 und beim Herausziehen aus der Kanüle 1 verwendbar ist. Zum anderen kann das Abschlusselement 15 dazu verwendet werden, die Kanüle 1, falls erforderlich, mit einem Eintriebsmittel, beispielsweise einem Hammer, an die gewünschte Position zu verbringen. Dazu ist das stirnseitige Ende des Abschlusselements 15 als Schlagfläche 16 ausgebildet. In der Mitte der Schlagfläche 16 ist eine proximale Mandrinöffnung 17 ausgebildet, die über einen sich über die gesamte Länge des Mandrins 14 in dessen Inneren erstreckenden Führungskanal 18 (siehe Fig. 2) mit einer am distalen Ende des Mandrins 14 vorgesehenen distalen Mandrinöffnung 19 verbunden ist. Der Führungskanal 18 sowie die beiden Mandrinöffnungen 17 und 19 sind dabei konzentrisch bezüglich der Längsachse des Mandrins 14 angeordnet.

In Fig. 2 ist die Kanüle nach Fig. 1 um 90° um ihre Längsachse 5 gedreht in aufgeschnittenem Zustand dargestellt. Insbesondere ist in Fig. 2 zu erkennen, dass der Mandrin 14 längsverschieblich jedoch im Wesentlichen spielfrei in dem Hohlraum 10 der Kanüle 1 angeordnet ist. Weiterhin ist zu erkennen, dass sich der Hohlraum 10 an seinem proximalen Ende verbreitert, um auf diese Weise das distale Ende des Abschlusselements 15 aufnehmen zu können.

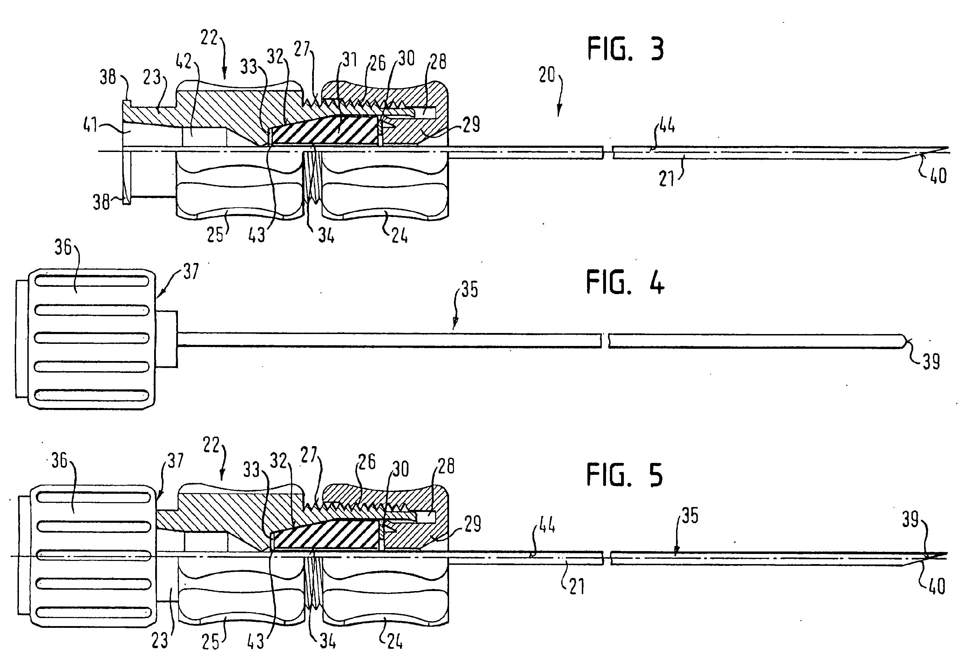

Fig. 3 zeigt eine erfindungsgemäß ausgebildete Injektionskanüle 20 in einem teilweise aufgerissenen Zustand. Die Injektionskanüle 20 umfasst eine Injektionsnadel 21, die als Hohlnadel ausgebildet ist, sowie ein an dem proximalen Ende der Injektionsnadel 21 befestigtes Anschlusselement 22 mit einem Anschlussstutzen 23. Der Anschlussstutzen 23 besitzt an seinem proximalen Ende radial nach außen ragende Ansätze 38, über die ein Anschluss einer Injektionsspritze bzw. eines Injektionskanülenmandrins (siehe Fig. 4 bis 7) möglich ist.

An der proximalen Stirnseite des Anschlussstutzens 23 ist eine spritzenseitige Anschlussöffnung 41 ausgebildete, die über einen in dem proximalen Teil 25 ausgebildeten Hohlraum 42 mit einer spritzenseitigen Öffnung 43 der Injektionsnadel 21 verbunden ist. Die spritzenseitige Öffnung 43 der Injektionsnadel 21 ist wiederum über einen im Inneren der Injektionsnadel 21 verlaufenden Injektionskanal 44 mit einer Injektionsöffnung 40 am distalen Ende der Injektionsnadel 21 verbunden.

Das Anschlusselement 22 umfasst ein distales Teil 24 und ein proximales Teil 25, die über ein Gewinde 26 miteinander verschraubt sind. Das proximale Teil 25 besitzt dazu einen zylinderförmigen Ansatz 27, der mit einem Außengewinde versehen ist und mit einem in einem Hohlraum 28 des distalen Teils 24 vorgesehenen Innengewinde zusammenwirkt.

In den Hohlraum 28 ragt ein Ansatz 29 des distalen Teils 24 hinein, der über einen Pressring 30 bei miteinander verschraubten Teilen 24, 25 auf die Stirnfläche eines aus elastischem Material, beispielsweise aus Gummi hergestellten Klemmelements 31 drückt. Das Klemmelement 31 ist sich in proximaler Richtung verjüngend ausgebildet und liegt mit seiner konischen Außenfläche 32 an einer ebenfalls schräg verlaufenden Innenfläche eines innerhalb des zylindrischen Ansatzes 27 und des proximalen Teils 25 ausgebildeten Hohlraums 33 an. Bei einem Verschrauben der beiden Teile 24 und 25 miteinander wird somit das Klemmelement 31 über den Ansatz 29 und den Pressring 30 so in proximaler Richtung verschoben, dass die konische Außenfläche 32 gegen die Gegenflächen des proximalen Teils 25 anlaufen, so dass das Klemmelement 31 in radialer Richtung zusammengepresst wird. Auf diese Weise wird eine Klemmkraft auf die Injektionsnadel 21, die durch eine Zentralbohrung 34 des Klemmelements geführt wird, erzeugt, so dass die Injektionsnadel 21 und das Anschlusselement 22 fest miteinander verbunden sind.

Fig. 4 zeigt einen Injektionskanülenmandrin 35, der an seinem proximalen Ende eine knopfförmige Handhabe 36 besitzt. Die Handhabe 36 besitzt einen zu ihrer distalen Seite hin offenen Hohlraum 37, dessen Außenwände mit einem nicht gezeigten Innengewinde versehen sind. Über dieses Innengewinde kann die Handhabe 36 mit den an dem Anschlussstutzen 23 des Anschlusselements 22 vorgesehenen Ansätzen 38 verschraubt werden.

Die distale Spitze 39 des Injektionskanülenmandrins 35 ist halbkugelförmig ausgebildet, wie aus Fig. 4 zu erkennen ist. Die Länge des Injektionskanülenmandrins 35 ist so gewählt, dass bei vollständig in die Injektionskanüle 20 eingesetztem Injektionskanülenmandrin 35 die abgerundete Spitze 39 des Injektionskanülenmandrins 35 in Höhe der Injektionsöffnung 40 der Injektionsnadel 21 zu liegen kommt und diese damit verschließt. Dadurch ist gewährleistet, dass beim Einführen der Injektionskanüle 20 kein Gewebe in die Injektionsnadel 21 eindringt. Dies ist aus Fig. 5 zu erkennen, die den zusammengebauten Zustand von Injektionskanüle 20 und Injektionskanülenmandrin 35 darstellt.

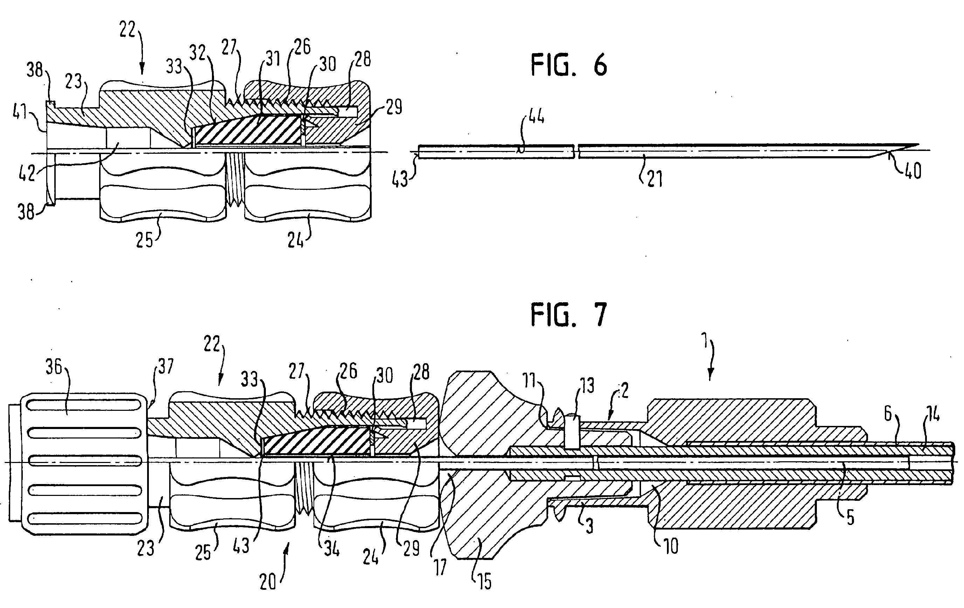

Fig. 6 zeigt den Zustand, wenn die Injektionsnadel 21 von dem Anschlusselement 22 getrennt ist. Es ist dabei zu erkennen, dass die Injektionsnadel 21 über ihre gesamte Länge eine gleichmäßige Querschnittsaußenabmessung besitzt, so dass ein Auffädeln der Kanüle 1 mit Hohlmandrin 14 auf das proximale Ende der Injektionsnadel 21 ermöglicht wird.

Fig. 7 zeigt letztlich die erfindungsgemäße Kanüle 1 mit der darin eingesetzten Injektionskanüle 20 und dem in diese wiederum eingesetzten Injektionskanülenmandrin 35.

Im Folgenden werden zwei mögliche Verwendungen der erfindungsgemäßen Kanüle beschrieben:
Zunächst wird die Injektionskanüle 20 mit eingesetztem Injektionskanülenmandrin 35, jedoch ohne aufgesetzte Injektionsspritze, relativ gering in das Gewebe eingestochen. Anschließend wird der Injektionskanülenmandrin 35 aus der Injektionskanüle 20 herausgezogen sowie das Anschlusselement 22 durch Verdrehen der beiden Teile 24 und 25 gegeneinander gelockert und von der Injektionsnadel 21 abgezogen, so dass das proximale Ende der Injektionsnadel 21 frei liegt.

Über dieses freie Ende kann dann die Applikationskanüle 1 zusammen mit dem eingesetzten Mandrin 14 über dessen distale Mandrinöffnung 19 aufgeschoben werden, bis das proximale Ende der Injektionsnadel 21 aus der proximalen Mandrinöffnung 17 herausragt. Anschließend wird das Anschlusselement 22 wieder auf dieses herausragende proximale Ende der Injektionsnadel 21 aufgesetzt und fixiert, wonach eine Injektionsspritze mit dem Anschlusselement 22 verbunden wird.

Über die Injektionsspritze wird Anästhesiemittel eingespritzt, so dass aus der Injektionsöffnung 40 der Injektionsnadel 21 Anästhesiemittel austritt und dadurch im Bereich der distalen Enden der Kanüle 1 sowie der Injektionskanüle 20 eine örtliche Betäubung erfolgt. Die beiden Kanülen 1 und 20 werden nun zusammen ständig weiter eingeschoben, wobei durch ständiges Abgeben von Anästhesiemittel eine Betäubung des jeweils gerade die distalen Enden der beiden Kanülen 1 und 20 umgebenden Gewebebereichs erfolgt. Das Einführen der beiden Kanülen 1 und 20 erfolgt solange zusammen, bis mit den distalen Enden der Knochen erreicht wird.

Anschließend wird die Injektionskanüle 20 aus dem Führungskanal 18 des Mandrins 14 herausgezogen und die Kanüle 1 zusammen mit dem noch eingesetzten Mandrin 14 in üblicher Weise in den Knochen eingeschlagen.

Nach erfolgter Positionierung wird dann der Mandrin 14 aus der Kanüle 1 herausgezogen und eine Applikationsvorrichtung mit dem Kopplungsabschnitt 3 der Kanüle 1 verbunden, über die dann in üblicher Weise beispielsweise Knochenzement appliziert wird.

Grundsätzlich ist es auch möglich, dass zunächst die Injektionskanüle 20 unter ständigem Einspritzen von Anästhesiemittel über eine angeflanschte Injektionsspritze ohne Kanüle 1 vollständig bis an die Cortex des Knochens eingeführt wird. Anschließend wird nach Abnahme der Spritze und Entfernen des Anschlusselements 22 die Kanüle 1 mit dem hohlen Mandrin 14 auf das aus dem Gewebe herausragende proximale Ende der Injektionsnadel 21 aufgefädelt und über die bereits platzierte Injektionsnadel 21 bis an die Cortex des Knochens herangeführt.

Da beim Einsetzen der Injektionsnadel 21 bereits kontinuierlich Anästhesiemittel eingespritzt wurde, ist der gesamte Einführbereich des Gewebes entlang der Injektionsnadel 21 betäubt, so dass das nachträgliche Einführen der Kanüle 1 keine Schmerzen verursacht.

Nachdem die Kanüle 1 vollständig eingeführt ist, kann dann die Injektionsnadel 21 wieder entfernt werden und die Kanüle 1 zusammen mit dem Mandrin 14 in üblicher Weise in den Knochen eingeschlagen werden. Anschließend wird dann der Mandrin 14 entfernt und nach Aufsetzen einer Applikationsvorrichtung der Knochenzement in den Knochen eingebracht.

### Bezugszeichenliste

- 1: Kanüle
- 2: proximales Ende
- 3: Kopplungsabschnitt
- 4: Betätigungselemente
- 5: Längsachse
- 6: Kanülenrohr
- 7: Spitze
- 8: distales Ende
- 9: distale Kanülenöffnung
- 10: Hohlraum
- 11: proximale Kanülenöffnung
- 12: L-förmige Ausnehmung
- 13: stiftförmiger Ansatz
- 14: Mandrin
- 15: Abschlusselement
- 16: Schlagfläche
- 17: proximale Mandrinöffnung
- 18: Führungskanal
- 19: distale Mandrinöffnung
- 20: Injektionskanüle
- 21: Injektionsnadel
- 22: Anschlusselement
- 23: Anschlussstutzen
- 24: distales Teil
- 25: proximales Teil
- 26: Gewinde
- 27: zylinderförmiger Ansatz
- 28: Hohlraum
- 29: Ansatz
- 30: Pressring
- 31: Klemmelement
- 32: konische Außenfläche
- 33: Hohlraum
- 34: Zentralbohrung
- 35: Injektionskanülenmandrin
- 36: Handhabe
- 37: Hohlraum
- 38: Ansatz
- 39: Spitze
- 40: Injektionsöffnung
- 41: Anschlussöffnung
- 42: Hohlraum
- 43: spritzenseitige Öffnung
- 44: Injektionskanal

## Patentansprüche

1. Knochenzement-Applikationssystem umfassend eine Kanüle mit einem innen liegenden Hohlraum (10), der sich von einer am distalen Ende (8) der Kanüle (1) ausgebildeten distalen Kanülenöffnung (9) zu einer am proximalen Ende (2) der Kanüle (1) ausgebildeten proximalen Kanülenöffnung (11) erstreckt, sowie mit einem Mandrin (14), der in dem Hohlraum (10) längsverschiebbar angeordnet ist,
**dadurch gekennzeichnet,**
**dass** der Mandrin (14) zur Aufnahme einer Injektionskanüle (20) einen innenliegenden Führungskanal (18) besitzt, der sich von einer am distalen Ende des Mandrins (14) ausgebildeten distalen Mandrinöffnung (19) zu einer am proximalen Ende des Mandrins (14) ausgebildeten proximalen Mandrinöffnung (17) erstreckt, und
**dass** eine Injektionskanüle (20) für eine Injektionsspritze vorgesehen ist, mit einer als Hohlnadel ausgebildeten Injektionsnadel (21), die einen innen liegenden Injektionskanal (44) umfasst, der sich von einer am distalen Ende der Injektionsnadel (21) ausgebildeten Injektionsöffnung (40) zu einer am proximalen Ende der Injektionsnadel (21) ausgebildeten spritzenseitigen Öffnung (43) erstreckt, wobei an dem proximalen Ende der Injektionsnadel (21) ein Anschlusselement (22) zum Anschließen der Injektionskanüle (20) an die Injektionsspritze befestigt ist, und wobei das Anschlusselement (22) von der Injektionsnadel (21) abnehmbar und an dieser wieder befestigbar ausgebildet ist.

2. Applikationssystem nach Anspruch 1,
**dadurch gekennzeichnet ,**
**dass** an dem proximalen Ende des Mandrins (14) ein Abschlusselement (15) ausgebildet ist, dass die proximale Mandrinöffnung (17) in dem Abschlusselement (15) ausgebildet ist und dass der Führungskanal (18) durch das Abschlusselement (15) hindurch zu der proximalen Mandrinöffnung (17) verläuft.

3. Applikationssystem nach einem der vorhergehenden Ansprüche,
**dadurch gekennzeichnet ,**
**dass** der Mandrin (14) in dem Hohlraum (10) im Wesentlichen spielfrei längsverschiebbar ist.

4. Applikationssystem nach einem der vorhergehenden Ansprüche,
**dadurch gekennzeichnet,**
**dass** der Hohlraum (10) einen im Wesentlichen kreisförmigen Querschnitt besitzt.

5. Applikationssystem, nach einem der vorhergehenden Ansprüche,
**dadurch gekennzeichnet ,**
**dass** der Führungskanal (18) einen im Wesentlichen kreisförmigen Querschnitt besitzt.

6. Applikationssystem, nach einem der vorhergehenden Ansprüche,
**dadurch gekennzeichnet**
**dass** an dem proximalen Ende (2) der Kanüle (1) ein Kopplungsabschnitt (3) zum Ankoppeln der Kanüle (1) an die Vorrichtung zum Applizieren von medizinischen Stoffen vorgesehen ist.

7. Applikationssystem nach einem der vorhergehenden Ansprüche,
**dadurch gekennzeichnet ,**
**dass** in dem Bereich des proximalen Endes (2) der Kanüle (1), insbesondere an dem Kopplungsabschnitt (3), ein Verbindungselement (12) zur Erzeugung einer lösbaren, drehfesten und/oder in axialer Richtung verschiebefesten Verbindung zwischen dem Mandrin (14) und der Kanüle (1) vorgesehenen ist.

8. Applikationssystem nach Anspruch 7,
**dadurch gekennzeichnet,**
**dass** an dem Mandrin (14), insbesondere im Bereich des proximalen Endes des Mandrins (14), ein mit dem Verbindungselement (12) zusammenwirkendes Gegenelement (13) vorgesehen ist.

9. Applikationssystem nach Anspruch 7 oder 8,
**dadurch gekennzeichnet ,**
**dass** die Verbindung zwischen dem Mandrin (14 und der Kanüle (1) durch einen Bajonettverschluß gebildet wird.

10. Applikationssystem, nach einem der Ansprüche 7 bis 9,
**dadurch gekennzeichnet ,**
**dass** das Verbindungselement als schlitzförmige Ausnehmung (12), insbesondere als Nut oder Durchbrechung, und das Gegenelement
als insbesondere stiftförmiger Ansatz (13) oder umgekehrt ausgebildet sind.

11. Applikationssystem, nach Anspruch 10,
**dadurch gekennzeichnet ,**
**dass** die Ausnehmung (12) zumindest einen in axialer Richtung der Kanüle (1) verlaufenden Längsabschnitt umfasst.

12. Applikationssystem nach Anspruch 11,
**dadurch gekennzeichnet ,**
**dass** sich an den Längsabschnitt ein in Umfangsrichtung der Kanüle (1) verlaufender Querabschnitt der Ausnehmung (12) anschließt.

13. Applikationssystem, nach einem der vorhergehenden Ansprüche,
**dadurch gekennzeichnet. ,**
**dass** das Anschlusselement (22) eine spritzenseitige Anschlussöffnung (41) umfasst, die bei befestigtem Anschlusselement (22) mit dem Injektionskanal (44) verbunden ist.

14. Applikationssystem, nach einem der vorhergehenden Ansprüche,
**dadurch gekennzeichnet,**
**dass** bei abgenommenem Anschlusselement (22) die Querschnittsaußenabmessung des proximalen Bereichs der Injektionsnadel (21)
im Wesentlichen gleich der Querschnittsaußenabmessung des übrigen Bereichs der Injektionsnadel (21) ist.

15. Applikationssystem nach einem der vorhergehenden Anspruche,
**dadurch gekennzeichnet,**
**dass** das Anschlusselement (22) eine Klemmeinheit (29, 30, 31, 32) zur klemmbaren Befestigung des Anschlusselements (22) an der Injektionsnadel (21) umfasst.

16. Applikationssystem nach einem der vorhergehenden Ansprüche,
**dadurch gekennzeichnet ,**
**dass** in den Injektionskanal (44) ein Injektionskanülenmandrin (35) einsetzbar ist.

17. Applikationssystem nach Anspruch 16,
**dadurch gekennzeichnet,**
**dass** der Injektionskanülenmandrin (35) in dem Injektionskanal (21) längsverschiebbar ist.

18. Applikationssystem, nach einem der Ansprüche 16 oder 17,
**dadurch gekennzeichnet,**
**dass** das distale Ende (39) des Injektionskanülenmandrins (35) abgerundet, insbesondere halbkugelförmig ausgebildet ist.

19. Applikationssystem nach einem der Ansprüche 16, bis 18,
**dadurch gekennzeichnet,**
**dass** der Injektionskanülenmandrin (35) so lang ausgebildet ist, dass bei vollständig in den Injektionskanal (44) eingesetztem Injektionskanülenmandrin (35) dessen distales Ende (39) in Höhe der Injektionsöffnung (40) zu liegen kommt.

20. Applikationssystem, nach einem der Ansprüche 16, bis 19,
**dadurch gekennzeichnet,**
**dass** an dem proximalen Ende (39) des Injektionskanülenmandrins (35) ein Fixierelement (36) vorgesehen ist, mit dem der Injektionskanülenmandrin (35) an der Injektionskanüle (20) in Längsrichtung unverschiebbar fixierbar ist.

21. Applikationssystem nach Anspruch 20,
**dadurch gekennzeichnet,**
**dass** das Fixierelement (36) an dem Anschlusselement (22) fixierbar, insbesondere mit diesem verschraubbar ist.

22. Applikationssystem nach einem der vorhergehenden Ansprüche,
**dadurch gekennzeichnet ,**
**dass** die Injektionskanüle (20) im Wesentlichen spielfrei längsverschiebbar in dem Führungskanal (18) des Mandrins (14) angeordnet ist.

## Claims

1. A bone cement application system having a cannula with an inwardly disposed hollow space (10) extending from a distal cannula opening (9) formed at the distal end (8) of the cannula (1) to a proximal cannula opening (11) formed at the proximal end (2) of the cannula (1) as well as having a mandrel (14) arranged longitudinally displaceably in the hollow space (10),
**characterised in that**
the mandrel (14) has an inwardly disposed guide passage (18) for the reception of an injection cannula (20), said guide passage extending from a distal mandrel opening (19) formed at the distal end of the mandrel (14) to a proximal mandrel opening (17) formed at the proximal end of the mandrel (14); and
**in that** an injection cannula (20) for an injection syringe is provided, comprising an injection needle (21) formed as a hollow needle which includes in inwardly disposed injection passage (44) which extends from an injection opening (40) formed at the distal end of the injection needle (21) to a syringe-side opening (43) formed at the proximal end of the injection needle (21), with a connection element (22) for the connection of the injection cannula (20) to the injection syringe being fastened at the proximal end of the injection needle (21) and with the connection element (22) being removable from the injection needle (21) and being fastenable to it again.

2. An application system in accordance with claim 1, **characterised in that** a terminal element (15) is formed at the proximal end of the mandrel (14); **in that** the proximal mandrel opening (17) is formed in the terminal element (15); and **in that** the guide passage (18) extends through the terminal element (15) to the proximal mandrel opening (17).

3. An application system in accordance with any one of the preceding claims, **characterised in that** the mandrel (14) is longitudinally displaceable substantially without play in the hollow space (10).

4. An application system in accordance with any one of the preceding claims, **characterised in that** the hollow space (10) has a substantially circular cross-section.

5. An application system in accordance with any one of the preceding claims, **characterised in that** the guide passage (18) has a substantially circular cross-section.

6. An application system in accordance with any one of the preceding claims, **characterised in that** a coupling section (3) is provided at the proximal end (2) of the cannula (1) for the coupling of the cannula (1) to the apparatus for the application of medical substances.

7. An application system in accordance with any one of the preceding claims, **characterised in that** a connection element (12) for the generation of a releasable connection fixed against rotation and/or fixed against displacement in the axial direction is provided between the mandrel (14) and the cannula (1) in the region of the proximal end (2) of the cannula (1), in particular at the coupling section (3).

8. An application system in accordance with claim 7, **characterised in that** a counter element (13) cooperating with the connection element (12) is provided at the mandrel (14), in particular in the region of the proximal end of the mandrel (14).

9. An application system in accordance with claim 7 or claim 8, **characterised in that** the connection between the mandrel (14) and the cannula (1) is formed by a bayonet fastening.

10. An application system in accordance with any one of the claims 7 to 9, **characterised in that** the connection element is made as a slit-like cut-out (12), in particular as a groove or opening, and the counter-element is made as a nose (13), in particular a pin-shaped nose, or vice versa.

11. An application system in accordance with claim 10, **characterised in that** the cut-out (12) includes at least one longitudinal section extending in the axial direction of the cannula (1).

12. An application system in accordance with claim 11, **characterised in that** a transverse portion of the cut-out (12) extending in the peripheral direction of the cannula (1) adjoins the longitudinal section.

13. An application system in accordance with any one of the preceding claims, **characterised in that** the connection element (22) includes a connection opening (41) at the syringe side which is connected to the injection passage (44) when the connection element (22) is fastened.

14. An application system in accordance with any one of the preceding claims, **characterised in that** the external dimension of the cross-section of the proximal region of the injection needle (21) is substantially equal to the external dimension of the cross-section of the remaining region of the injection needle (21) when the connection element is removed.

15. An application system in accordance with any one of the preceding claims, **characterised in that** the connection element (22) includes a clamping unit (29, 30, 31, 32) for the clampable fastening of the connection element (22) to the injection needle (21).

16. An application system in accordance with any one of the preceding claims, **characterised in that** an injection cannula mandrel (35) is insertable into the injection passage (44).

17. An application system in accordance with claim 16, **characterised in that** the injection cannula mandrel (35) is longitudinally displaceable in the injection passage (21).

18. An application system in accordance with any one of the claims 16 or 17, **characterised in that** the distal end (39) of the injection cannula mandrel (35) is rounded, is in particular semi-spherical.

19. An application system in accordance with any one of the claims 16 to 18, **characterised in that** the injection cannula mandrel (35) is made so long that, when the injection cannula mandrel (35) is inserted into the injection channel (44), the former's distal end (39) comes to lie at the level of the injection opening (40).

20. An application system in accordance with any one of the claims 16 to 19, **characterised in that** a fixing element (36) with which the injection cannula mandrel (55) can be fixed to the injection channel (20) non-displaceably in the longitudinal direction is provided at the proximal end (39) of the injection cannula mandrel (35).

21. An application system in accordance with claim 20, **characterised in that** the fixing element (36) is fixable to the connection element (22), is in particular screwable thereto.

22. An application system in accordance with any one of the preceding claims, **characterised in that** the injection cannula (20) is arranged longitudinally displaceably substantially without play in the guide passage (18) of the mandrel (14).

## Revendications

1. Système d'application de ciment osseux, comprenant une canule avec une cavité (10) située à l'intérieur, laquelle s'étend depuis une ouverture de canule distale réalisée à l'extrémité distale (8) de la canule (1) jusqu'à une extrémité de canule proximale (11) réalisée à l'extrémité proximale (2) de la canule (1), et comprenant un mandrin (14) agencé en translation longitudinale dans la cavité (10),
**caractérisé en ce que**
le mandrin (14) possède un canal de guidage (18) situé à l'intérieur et destiné à recevoir une canule d'injection (20), lequel s'étend depuis une ouverture de mandrin distale (19) réalisée à l'extrémité distale du mandrin (14) jusqu'à une ouverture de mandrin proximale (17) réalisée à l'extrémité proximale du mandrin (14), et **en ce qu'**il est prévu une canule d'injection (20) pour une seringue d'injection, dotée d'une aiguille d'injection (21) réalisée sous forme d'aiguille creuse qui présente un canal d'injection (44) situé à l'intérieur, lequel s'étend depuis une ouverture d'injection (40) ménagée à l'extrémité distale de l'aiguille d'injection (21) jusqu'à une ouverture (43) côté seringue réalisée à l'extrémité proximale de l'aiguille d'injection (21),
dans lequel à l'extrémité proximale de l'aiguille d'injection (21) est fixé un élément de raccordement (22) pour raccorder la canule d'injection (20) à la seringue d'injection, et
dans lequel l'élément de raccordement (22) est réalisé susceptible d'être enlevé de l'aiguille d'injection (21) et d'être de nouveau fixé à celle-ci.

2. Système d'application selon la revendication 1,
**caractérisé en ce qu'**un élément de fermeture (15) est réalisé à l'extrémité proximale du mandrin (14), **en ce que** l'ouverture de mandrin proximale (17) est réalisée dans l'élément de fermeture (15), et **en ce que** le canal de guidage (18) s'étend en traversant l'élément de fermeture (15) vers l'ouverture de mandrin proximale (17).

3. Système d'application selon l'une des revendications précédentes,
**caractérisé en ce que** le mandrin (14) est capable de translation longitudinale essentiellement sans jeu dans la cavité (10).

4. Système d'application selon l'une des revendications précédentes,
**caractérisé en ce que** la cavité (10) possède une section transversale essentiellement de forme circulaire.

5. Système d'application selon l'une des revendications précédentes,
**caractérisé en ce que** le canal de guidage (18) possède une section transversale essentiellement de forme circulaire.

6. Système d'application selon l'une des revendications précédentes,
**caractérisé en ce qu'**il est prévu à l'extrémité proximale (2) de la canule (1) un tronçon d'accouplement (3) pour accoupler la canule (1) au dispositif destiné à appliquer des substances médicales.

7. Système d'application selon l'une des revendications précédentes,
**caractérisé en ce que**, dans la région de l'extrémité proximale (2) de la canule (1), en particulier au niveau du tronçon d'accouplement (3), il est prévu un élément de liaison (12) pour engendrer entre le mandrin (14) et la canule (1) une liaison détachable, solidaire en rotation et/ou solidaire en translation en direction axiale.

8. Système d'application selon la revendication 7,
**caractérisé en ce qu'**il est prévu sur le mandrin (14), en particulier dans la région de l'extrémité proximale du mandrin (14), un élément antagoniste (13) qui coopère avec l'élément de liaison (12).

9. Système d'application selon la revendication 7 ou 8,
**caractérisé en ce que** la liaison entre le mandrin (14) et la canule (1) est formée par une fermeture à baïonnette.

10. Système d'application selon l'une des revendications 7 à 9,
**caractérisé en ce que** l'élément de liaison est réalisé sous la forme d'un évidement (12) en forme de fente, en particulier en forme de gorge ou de traversée, et **en ce que** l'élément antagoniste est réalisé sous forme de talon (13) en particulier en forme de tige, ou vice versa.

11. Système d'application selon la revendication 10,
**caractérisé en ce que** l'évidement (12) comprend au moins un tronçon longitudinal qui s'étend en direction axiale de la canule (1).

12. Système d'application selon la revendication 11,
**caractérisé en ce qu'**un tronçon transversal de l'évidement (12), qui s'étend en direction périphérique de la canule (1), se raccorde au tronçon longitudinal.

13. Système d'application selon l'une des revendications précédentes,
**caractérisé en ce que** l'élément de raccordement (22) comprend une ouverture de raccordement (41) côté seringue, laquelle est reliée au canal d'injection (44) lorsque l'élément de raccordement (22) est fixé.

14. Système d'application selon l'une des revendications précédentes,
**caractérisé en ce que**, lorsque l'élément de raccordement (22) est enlevé, la dimension extérieure en section transversale de la région proximale de l'aiguille d'injection (21) est essentiellement égale à la dimension extérieure en section transversale de la région restante de l'aiguille d'injection (21).

15. Système d'application selon l'une des revendications précédentes,
**caractérisé en ce que** l'élément de raccordement (21) comprend une unité de serrage (29, 30, 31, 32) pour fixer par serrage l'élément de raccordement (22) sur l'aiguille d'injection (21).

16. Système d'application selon l'une des revendications précédentes,
**caractérisé en ce qu'**un mandrin de canule d'injection (35) est susceptible d'être mis en place dans le canal d'injection (44).

17. Système d'application selon la revendication 16,
**caractérisé en ce que** le mandrin de canule d'injection (35) est capable de translation longitudinale dans le canal d'injection (21).

18. Système d'application selon l'une des revendications 16 ou 17,
**caractérisé en ce que** l'extrémité distale (39) du mandrin de canule d'injection (35) est réalisé arrondi, en particulier en forme de demi-sphère.

19. Système d'application selon l'une des revendications 16 à 18,
**caractérisé en ce que** le mandrin de canule d'injection (35) est réalisé aussi long que, lorsque le mandrin de canule d'injection (35) est entièrement mis en place dans le canal d'injection (44), son extrémité distale (39) vient se placer à la hauteur de l'ouverture d'injection (40).

20. Système d'application selon l'une des revendications 16 à 19,
**caractérisé en ce qu'**il est prévu à l'extrémité proximale (39) du mandrin de canule d'injection (35) un élément de fixation (35) au moyen duquel le mandrin de canule d'injection (35) est susceptible d'être fixé sur la canule d'injection (20) sans possibilité de translation en direction longitudinale.

21. Système d'application selon la revendication 20,
**caractérisé en ce que** l'élément de fixation (36) est susceptible d'être fixé sur l'élément de raccordement (22), en particulier d'être vissé à celui-ci.

22. Système d'application selon l'une des revendications précédentes,
**caractérisé en ce que** la canule d'injection (20) est agencée avec possibilité de translation longitudinale essentiellement sans jeu dans le canal de guidage (18) du mandrin (14).
